# EUROPEAN PATENT SPECIFICATION

(11) **EP 2 124 978 B1**
(45) Date of publication and mention of the grant of the patent: **20.12.2017**
(21) Application number: 08780459.7
(22) Date of filing: 28.02.2008
(51) Int. Cl.: A61K 35/76, A61L 2/00

(54) **EXTERNAL ANIMAL LAYER SANITATION USING BACTERIOPHAGE**
TIERHYGIENE AN ÄUSSEREN SCHICHTEN UNTER VERWENDUNG VON BAKTERIOPHAGEN
ASSAINISSEMENT DE LA COUCHE EXTERNE D'UN ANIMAL À L'AIDE D'UN BACTÉRIOPHAGE

(30) Priority: 28.02.2007 US 904151 P
(43) Date of publication of application: 02.12.2009
(73) Proprietor: Omnilytics, Inc., Salt Lake City, UT 84116 (US)
(72) Inventor: REBER, Justin, Highland, Utah 84003 (US); JACKSON, Lee E., Layton, Utah 84040 (US)
(74) Representative: van Heuvel, Margaretha
(86) International application number: PCT/US2008/055344
(87) International publication number: WO 2008/127795

(56) References cited:
- WO-A1-03/067991
- WO-A2-01/50872
- SULAKVELIDZE ET AL.: 'Antimicrobial Agents and Chemotherapy' BACTERIOPHAGE THERAPY vol. 45, no. 3, March 2001, pages 649 - 659, XP008115592

## Description

### TECHNICAL FIELD

The present invention relates generally to methods for sanitizing external layers of animals and, more specifically, to the use of viruses, such as bacteriophage, to control populations of microorganisms, such as bacteria, on external layers of animals.

### BACKGROUND ART

The spread of pathogens from animal sources (*e.g.*, food, etc.) to humans is a significant cause of sickness and death in many parts of the world. Concerns over the health and economic consequences of animal-related illnesses have led to the widespread use of antibiotics and similar drugs-in both humans and animals. While antibiotics were initially very effective for treating illnesses caused by bacteria, and retained their effectiveness for a significant portion of modern history, their overuse has resulted in the emergence of antibiotic-resistance microorganisms.

Ongoing research efforts are aimed at developing alternative mechanisms for curbing the transmission of pathogens from animals to humans. For example, microbial contamination of animal carcasses has been experimentally controlled with viruses, such as bacteriophage. The application of bacteriophage or other viruses directly to meat that is intended for human and even animal consumption is, however, considered to be somewhat undesirable by many people.

Furthermore, these processes, to date, have been limited to the internal administration of phage and the application of phage to solid surfaces of animal products that are to be eaten, such as the surfaces of carcasses, see eg. WO 01/50872. The treatment of animal products that are used for other purposes (*e.g*., in the manufacture of clothing, furniture, etc.), has been largely ignored. Among these processes are processes for removing, obtaining and preserving external layers from animals. The removal and preservation of hides, skins, furs, pelts and other external layers from animals are often highly unsanitary processes. When the external animal layers are first obtained, regardless of the processes that will be used to preserve them, they may be contaminated with feces, urine, blood, animal fat, animal flesh, and other reservoirs for microbial, particularly bacterial, growth. In instances where ancient techniques are practiced, numerous additional opportunities for the proliferation and spread of bacteria and other microorganisms are presented. When ancient techniques are employed, individuals may repeatedly handle or otherwise contact the external animal layers throughout the preservation process, which itself presents numerous additional opportunities (*e.g.*, during de-hairing, bating, and pickling processes) for microorganisms to grow and spread. Ancient techniques for tanning hides or otherwise preserving external animal layers, including skins and furs, are still widely practiced, particularly by individuals and in less developed areas of the world. Even when modern processes for removing or preserving external animal layers are employed, individuals may be subjected to highly unsanitary conditions.

In addition to posing a threat to individuals who participate in the removal or preservation processes, microorganisms, such as bacteria, may also damage the external layer that is being preserved. In recognition of the potential for such damage, heat and/or biocides, such as pentachlorophenol and other chemicals, are used to prevent the growth of microorganism on external layers from animals. The use of heat and/or chemicals to remove microorganisms from external layers may, however, damage the external layers (e.g., the corium and/or grain layer).

### DISCLOSURE OF INVENTION

The present invention, in various embodiments, includes methods according to claim 1 in which viruses, such as bacteriophage, or mixtures of viruses are applied to the exterior, or external layer, of an animal. For the sake of simplicity, the term "phage" as used herein refers to bacteriophage and any other type of virus that specifically infects another microorganism. A phage that is exteriorly applied to an animal may be targeted toward undesirable microorganisms, such as harmful microorganisms or microorganisms that are otherwise unwanted (*e.g*., microorganisms that cause spoilage, decomposition, unpleasant odors or tastes, etc.). The phrase "external layer," as used herein, includes any of hide, pelt, skin, or rind, together with or separately from coverings thereof, such as wool, fur, hair, feathers, and scales.

The phage may be applied to the exterior of an animal after its death (*e.g*., by slaughter or otherwise), for example, before the animal's hide, or "external layer" is removed from other parts of the animal's body, such as its carcass.

The present invention also includes embodiments of methods in which phage is applied to an external layer of an animal as the external layer is removed from other parts of the animal's body.

In other embodiments, the present invention includes methods in which phage is applied to an external layer after it has been removed from the animal's carcass. In a more specific embodiment, phage may be applied to an animal hide before and/or during processes for preserving the animal hide.

Other aspects, as well as features and advantages, of the present invention will become apparent to those of ordinary skill in the art through consideration of the ensuing description and the appended claims.

### BEST MODE FOR CARRYING OUT INVENTION

There is believed to be some correlation between the presence of undesirable microorganisms, including pathogens, on a slaughtered animals' hide and contamination of the same animal's carcass by such undesirable microorganisms. It is believed that many of the undesirable microorganisms that find their way onto the hides of slaughtered animals come from that animal's own intestinal tract. Undesirable microorganisms from environments to which an animal is exposed (*e.g*., the environment in which the animal lived, such as a feed lot, a henhouse, on the range, etc., or to which the animal was otherwise exposed, such as transport vehicles, in a slaughterhouse, etc.) may also contaminate the animal's hide, and after the animal has been slaughtered, may ultimately contaminate its carcass.

The present invention includes methods according to claim 1 for preventing the transfer of undesirable microorganisms that are present on the exterior of an animal from spreading to humans and to other animals. The application of phage in accordance with embodiments of the present invention may result in a sufficient reduction in population of a sufficient number of one or more target microorganisms (*e.g*., a reduction of about one log, or about 90%, or more (*e.g*., up to a reduction of about 4 log, or 99.99%)) or a sufficient prevention of the expected growth of one or more target microorganisms (*e.g.*, a prevention of at least about 90%, or even of about 99.99%, of the expected growth) to prevent their undesired effects, or rendering the microorganisms more susceptible to other antimicrobial treatments, for example, to chemotherapeutic antibiotics (*e.g*., a broad spectrum antibiotic, a narrow spectrum antibiotic, such as bacteriocin nisin, etc.).

One embodiment of a composition that may be used to reduce populations of microorganisms and/or to prevent microorganisms from proliferating to their potential includes one or more types of lytic phage, which will infect and kill one or more undesirable targeted microorganisms (*e.g.,* bacteria, etc.). Another embodiment of composition that may be used in accordance with teachings of the present invention may include lysogenic phage, which incorporate their genomes into the genomes of their hosts and employ the gene expression, or protein production, mechanisms of their hosts to produce compounds, such as lysin enzymes, that will kill or inhibit the proliferation of one or more undesired target microorganisms. Embodiments of compositions that include both lytic and lysogenic phage may also be used in accordance with teachings of the present invention.

Phage that is used in accordance with teachings of the present invention may have host range that includes the wild-type of a target microorganism, as well as one or more phage-resistant mutants of the target microorganism, such as the so-called "h-mutant" lytic phage described in U.S. Patent Application Publication US-2006-0153811-A1, the entire disclosure of which is, by this reference, hereby incorporated herein.

The phage that are used in accordance with teachings of the present invention may also be selected, using known techniques, to survive under certain pH conditions (*e.g.,* highly acidic conditions, highly basic conditions, etc.), salt conditions, temperature variations, or the like, so that they may remain viable and useful under conditions in which targeted microorganisms may survive.

Examples of microorganisms that may be targeted by a phage of the composition include, but are not limited to, one or more strains of *Escherichia coli* (*e.g., E. coli* 0157:H7, including, but not limited to, "Pattern 15," etc.) and various species of bacteria within the genera *Salmonella* (*e.g., Salmonella enterica,* etc.), *Staphylococcus* (*e.g., Staphylococcus aureus, Staphylococcus epidermidis,* etc.), *Pseudomonas* (*e.g., Pseudomonas aeruginosa,* etc.), *Shigella, Campylobacter, Bacillus* (*e.g., Bacillus anthracis, Bacillus cereus,* etc.), *Hemophilus, Bordetella, Francisella, Brucella (e.g., Brucella abortus, Brucella suis, Brucella melitensis,* etc.), *Listeria, Yersinia, Streptococci,* and *Vancomycin-Resistant Enterococci* (VRE) (*e.g., E. faecium, E. faecalis, E. gallinarium,* etc.).

In some embodiments, phage may be included in a substantially cell-free composition. Phage in other embodiments may be provided within a carrier host (*e.g.,* within nonpathogenic host cells).

A composition that includes phage may be embodied in dry form or liquid form. An embodiment of a composition in dry, particulate or powdered form, may be manufactured by known processes, such as those disclosed in U.S. Patent Application Serial No. 60/976,727, the disclosure of which is hereby incorporated herein, in its entirety, by this reference. In addition to phage, a dry composition may include fillers. A dry form of a phage containing composition may be dusted or dry-sprayed onto an animal.

A liquid form of a composition according to another embodiment of the present invention includes phage in a solution that may also include ingredients that stabilize the phage during storage and transportation. A liquid form of a phage containing composition may be sprinkled or sprayed (*e.g.,* as a mist or fog, as a high pressure stream, etc.) onto the animal or the animal may be introduced into a bath of the composition in either a concentrated or diluted form.

In either phage application technique, phage may penetrate (*e.g.,* coat when a dry composition is used, soak when a liquid composition is used) the animal's outer coat (*e.g.,* wool, fur, hair, feathers, scales, etc.) to which the composition is applied. Phage may be applied to all areas of an animal's exterior, or merely to locations on the animal's exterior that are most likely to carry unwanted target microorganisms (*e.g.,* buttocks, feet, legs, etc.).

Phage may be applied once, or a number of times (*i.e.,* periodically).

In addition, in some embodiments, a chemotherapeutic antimicrobial agent, such as bacteriocin nisin, may be applied to the external layer, either in conjunction with or following application of phage to the external layer.

In one aspect, such a process may reduce or eliminate the transmission of one or more undesirable targeted microorganisms to an animal's carcass and, ultimately, to food products derived from the animal's carcass is reduced or eliminated.

In embodiments where phage is applied to the exterior of an animal before external layers of the animal are removed from its carcass, the techniques (*e.g.,* application under high pressure, soaking, etc.) by which phage is applied may also be configured to remove reservoirs (*e.g.,* feces, urine, soil, etc.) for microorganisms, such as *Shigella* and shiga toxin-producing *E. coli,* from the exterior of the animal. In this regard, the application process may be conducted as part of a washing or sanitation process (*e.g.,* with a surfactant, wetting-agent, soap or other chemical sanitizer, etc.). In alternative embodiments, potential reservoirs for microorganisms may be removed from exterior locations of an animal by conventional washing processes before phage is applied to at least portions of the exterior of an animal.

The application of phage and any washing may occur after the animal has been slaughtered. Measures may be taken following the application of phage to minimize its removal from the animal's exterior and, optionally, to minimize exposure of the exterior of the animal to more unwanted microorganisms.

Once the animal has been slaughtered, an external layer or portion of an external layer to which phage has been applied may be removed from another portion of the animal's body (*e.g.,* its carcass, etc.). With populations of one or more unwanted microorganisms minimized, or at least exposed to phage that will lyse the same, the external layer may be removed with a reduced risk that any unwanted target microorganism thereon will be transferred to, or contaminate, and proliferate on the animal's carcass or any another portion of the animal's body. Additionally, the likelihood that unwanted target microorganisms will be transferred to and proliferate on surfaces in environments where external layers are removed and animal products are subsequently processed will be reduced or minimized.

In other embodiments, the transmission of microorganisms from an animal's external layer to its carcass may be prevented by applying phage to the external layer during its removal from other parts of the animal's body, such as its carcass.

Such application may be effected by spraying, dusting, soaking, or in any other suitable manner, depending at least in part, of course, upon the form (*e.g.,* dry, liquid, etc.) of phage containing composition applied to the external layer. As in embodiments where phage is applied before removal of an external layer from an animal, phage may be applied as part of or following a wash or sanitation process.

In some embodiments, phage may be concurrently applied to the external layer and to the parts of the animal from which the external layer is removed. The application of phage in this manner prevents any target microorganisms that may be transferred from the external layer to other parts of the animal from growing on the other parts. The application of phage concurrently to an external layer and to the parts of an animal from which an external layer is removed may also increase the efficiencies and economics associated with phage application processes, as a single phage application process may replace multiple processes.

Another aspect of the present invention includes application of phage to an external layer of an animal following removal of the external layer from other parts of the animal's body. In such embodiments, phage may be applied to both the exterior surface of the external layer and the previously internal surface of the exterior layer.

Such application may be effected by spraying, dusting, soaking, or in any other suitable manner, depending at least in part, of course, upon the form (*e.g.,* dry, liquid, etc.) of phage containing composition applied to the external layer. As in embodiments where phage is applied before or during removal of an external layer from an animal, phage may be applied as part of or following a wash or sanitation process.

Embodiments in which phage is applied to an external layer during or after its removal from other parts of an animal's body are particularly useful when the external layer is to be handled or processed and, during such handling or processing, may present a risk for the transmission of one or more unwanted microorganisms. Examples of such processing include, but are not limited to, processes by which external animal layers are processed, such as wool and other hairs that are shorn from animals, or preserved, such as in tanning processes and processes for preserving furs.

In an embodiment of an external layer preservation method according to the present invention, an external layer may be exposed to a composition that includes phage at one or more points throughout the tanning process. For example, phage may be applied to an external layer shortly after its removal from the remainder of the body of an animal, before the external layer is cured. The application of phage prior to curing may prevent any damage that microorganisms may cause between the time the external layer is removed from the remainder of an animal body and the time at which the preservation process actually begins. As another example, an external layer may be soaked in a phage-containing solution following and during the curing process (and, in leather making processes, following the removal of hair from the external layer), but before the external layer is exposed to tanning agents, another point in the process at which the external layer may be susceptible to microbial infection. Phage may also be applied to an external layer during or after the completion of preservation processes.

In another aspect, the present invention includes compositions for preventing microbial growth during the preservation of external layers for animals. Such a composition includes, and may consist essentially of, phage against at least one targeted microorganism. The phage may be selected to withstand conditions (*e.g*., pH, saltiness, extreme temperatures, etc.) of a process *(e.g.,* external layer treatment, external layer preservation, etc.) in which it may be used. Of course, in some embodiments, a preservation composition may also include buffers and other ingredients that may be useful in some part of the overall preservation process.

Although the foregoing description contains many specifics, these should not be construed as limiting the scope of the present invention, but merely as providing illustrations of some embodiments. Similarly, other embodiments of the invention may be devised which do not exceed the scope of the present invention. Features from different embodiments may be employed in combination. The scope of the invention is, therefore, indicated and limited only by the appended claims rather than by the foregoing description. All additions, deletions and modifications to the invention as disclosed herein which fall within the meaning and scope of the claims are to be embraced thereby.

## Claims

1. A method for sanitizing an animal product, comprising:
applying phage targeted for at least one type of microorganism to an external layer of a
body of an animal that has been slaughtered, and to normal coverings of the external layer; and
after the animal has been slaughtered, and before applying the phage, while applying
the phage or after applying the phage, removing the external layer from a remainder of the body of the animal, the phage on the external layer and the normal coverings of the external layer minimizing contamination by microorganisms on the external layer of the animal or preventing transmission of microorganisms from the external layer to a carcass of the body of the animal while removing the external layer.

2. The method of claim 1, wherein applying comprises applying at least one of a dry composition including the phage and a liquid composition including the phage to the external layer.

3. The method of claim 1, wherein applying comprises penetrating a coating on the external layer.

4. The method of claim 3, wherein applying comprises applying phage to the external layer under pressure.

5. The method of claim 1, wherein applying comprises applying the phage to the external layer of the animal before removing the external layer from the remainder of the body of the animal or after the external layer has been removed from the remainder of the body of the animal.

6. The method of claim 1, wherein applying comprises applying the phage to the external layer of the animal while removing the external layer from the remainder of the body of the animal.

7. The method of claim 6, wherein applying comprises applying the phage to exterior and interior surfaces of the external layer.

8. The method of claim 7, wherein applying further comprises applying the phage to the remainder of the body of the animal.

9. The method of claim 1, comprising washing or sanitizing the external layer.

10. The method of claim 9, wherein washing or sanitizing is effected before applying or washing or sanitizing is effected concurrently with applying.

11. The method of claim 9, wherein washing or sanitizing includes removing at least one reservoir for growth of at least one microorganism from the external layer.

12. The method of claim 1, wherein applying is effected as part of a process for preserving the external layer.

13. The method of claim 12, wherein applying comprises applying phage selected to withstand at least one condition of the process for preserving the external layer.

14. The method of claim 1, wherein applying comprises applying at least one of a lytic phage and a lysogenic phage.

15. The method of claim 1, wherein applying comprises applying a lytic phage.

## Patentansprüche

1. Verfahren zum keimfrei machen eines tierischen Produkts, das umfasst:
das Aufbringen eines Phagen, der mindestens gegen einen Typ eines Mikroorganismus gerichtet ist, auf eine äußere Schicht eines Tierkörpers, der geschlachtet wurde, und auf normale Außenhüllen der äußeren Schicht; und
das Entfernen der äußeren Schicht von dem Rest des Tierkörpers, des Phagen auf der äußeren Schicht und der normalen Außenhüllen der äußeren Schicht, nachdem das Tier geschlachtet wurde und bevor der Phage aufgebracht wird, während der Phage oder nachdem der Phage aufgebracht wird, was die Verunreinigung durch Mikroorganismen auf der äußeren Schicht des Tieres minimiert, oder was eine Übertragung von Mikroorganismen von der äußeren Schicht auf eine Karkasse des Tierkörpers während des Entfernens der äußeren Schicht verhindert.

2. Verfahren nach Anspruch 1, wobei das Aufbringen das Aufbringen von mindestens einer trockenen Zusammensetzung umfasst, die den Phagen und eine flüssige Zusammensetzung beinhaltet, die den Phagen der äußeren Schicht beinhaltet.

3. Verfahren nach Anspruch 1, wobei das Aufbringen das Eindringen eines Belags in die äußere Schicht umfasst.

4. Verfahren nach Anspruch 3, wobei das Aufbringen das Aufbringen des Phagen auf die äußere Schicht unter Druck umfasst.

5. Verfahren nach Anspruch 1, wobei das Aufbringen das Aufbringen des Phagen auf die äußere Schicht des Tieres umfasst, bevor die äußere Schicht von dem Rest des Tierkörpers oder nachdem die äußere Schicht von dem Rest des Tierkörpers entfernt wurde.

6. Verfahren nach Anspruch 1, wobei das Aufbringen das Aufbringen des Phagen auf die äußere Schicht des Tieres umfasst, während die äußere Schicht von dem Rest des Tierkörpers entfernt wird.

7. Verfahren nach Anspruch 6, wobei das Aufbringen das Aufbringen des Phagen auf die Außenflächen und die Innenflächen der äußeren Schicht umfasst.

8. Verfahren nach Anspruch 7, wobei das Aufbringen zudem das Aufbringen des Phagen auf den Rest des Tierkörpers umfasst.

9. Verfahren nach Anspruch 1, das das Waschen oder keimfrei machen der äußeren Schicht umfasst.

10. Verfahren nach Anspruch 9, wobei das Waschen oder keimfrei machen vor dem Aufbringen erfolgt, oder wobei das Waschen oder keimfrei machen gleichzeitig mit dem Aufbringen erfolgt.

11. Verfahren nach Anspruch 9, wobei das Waschen oder keimfrei machen das Entfernen von mindestens einem Reservoir für das Wachstum von mindestens einem Mikroorganismus der äußeren Schicht beinhaltet.

12. Verfahren nach Anspruch 1, wobei das Aufbringen als Teil eines Verfahrens für die Konservierung der äußeren Schicht erfolgt.

13. Verfahren nach Anspruch 12, wobei das Aufbringen das Aufbringen des Phagen umfasst, der ausgewählt ist, um mindestens einer Bedingung des Verfahrens zur Konservierung der äußeren Schicht standzuhalten.

14. Verfahren nach Anspruch 1, wobei das Aufbringen das Aufbringen mindestens eines lytischen Phagen und eines lysogenen Phagen umfasst.

15. Verfahren nach Anspruch 1, wobei das Aufbringen das Aufbringen eines lytischen Phagen umfasst.

## Revendications

1. Procédé d'assainissement d'un produit animal, comprenant :
l'application d'un phage ciblé pour au moins un type de micro-organisme à une couche externe d'un corps d'un animal qui a été abattu, et à des enrobages normaux de la couche externe ; et
après que l'animal a été abattu, et avant application du phage, tout en appliquant le phage ou après application du phage, l'élimination de la couche externe d'un reste du corps de l'animal, de phage sur la couche externe et des enrobages normaux de la couche externe minimisant la contamination par des micro-organismes sur la couche externe de l'animal ou empêchant la transmission de micro-organismes de la couche externe à une carcasse du corps de l'animal tout en éliminant la couche externe.

2. Procédé selon la revendication 1, dans lequel l'application comprend l'application d'au moins l'une d'une composition sèche incluant le phage et d'une composition liquide incluant le phage à la couche externe.

3. Procédé selon la revendication 1, dans lequel l'application comprend la pénétration d'un revêtement sur la couche externe.

4. Procédé selon la revendication 3, dans lequel l'application comprend l'application d'un phage à la couche externe sous pression.

5. Procédé selon la revendication 1, dans lequel l'application comprend l'application du phage à la couche externe de l'animal avant d'éliminer la couche externe du reste du corps de l'animal ou après que la couche externe a été éliminée du reste du corps de l'animal.

6. Procédé selon la revendication 1, dans lequel l'application comprend l'application du phage à la couche externe de l'animal tout en éliminant la couche externe du reste du corps de l'animal.

7. Procédé selon la revendication 6, dans lequel l'application comprend l'application du phage à des surfaces extérieure et intérieure de la couche externe.

8. Procédé selon la revendication 7, dans lequel l'application comprend en outre l'application du phage au reste du corps de l'animal.

9. Procédé selon la revendication 1, comprenant le lavage et l'assainissement de la couche externe.

10. Procédé selon la revendication 9, dans lequel le lavage ou l'assainissement est effectué avant que l'application ou le lavage ou l'assainissement soit effectué simultanément à l'application.

11. Procédé selon la revendication 9, dans lequel le lavage ou l'assainissement inclut l'élimination d'au moins un réservoir de croissance d'au moins un micro-organisme depuis la couche externe.

12. Procédé selon la revendication 1, dans lequel l'application est effectuée en tant que partie d'un processus de préservation de la couche externe.

13. Procédé selon la revendication 12, dans lequel l'application comprend l'application d'un phage choisi pour supporter au moins une condition du processus de préservation de la couche externe.

14. Procédé selon la revendication 1, dans lequel l'application comprend l'application d'au moins l'un d'un phage lytique et d'un phage lysogène.

15. Procédé selon la revendication 1, dans lequel l'application comprend l'application d'un phage lytique.
